# EUROPEAN PATENT APPLICATION

(11) **EP 4 585 676 A1**
(43) Date of publication of application: **16.07.2025**
(21) Application number: 23863126.1
(22) Date of filing: 04.09.2023
(51) Int. Cl.: C12M 3/00, C12N 5/071, C12Q 1/02, G01N 1/36

(54) **BLOCK FOR PREPARING OBSERVATION SECTION, AND USE THEREOF**

(30) Priority: 05.09.2022 JP 2022140997
(71) Applicant: RIKEN, Wako-shi, Saitama 351-0198 (JP)
(72) Inventor: HAGIWARA, Masaya, Wako-shi, Saitama 351-0198 (JP); KOH, Isabel, Siew Yin, Wako-shi, Saitama 351-0198 (JP)
(74) Representative: Graf von Stosch Patentanwaltsgesellschaft mbH
(86) International application number: PCT/JP2023/032166
(87) International publication number: WO 2024/053588

(57) **Abstract**

A block for preparation of an observation section in accordance with the present disclosure includes: a cell aggregate that has a three-dimensional shape and that is embedded; and a marker that is continuous or intermittent and that extends in a predetermined direction.

## Description

### Technical Field

The present invention relates to, for example, a block for preparation of an observation section, a method for preparing an observation section, an observation section, and a method for observing an observation section.

### Background Art

Studies for forming vascular tissues, bronchial tissues, etc. by three-dimensionally culturing cell tissues in culture gels have been carried out (see, for example, Patent Literatures 1 to 3). In these studies, a thick culture gel layer is ordinarily formed in a dish or well, and cells or cell tissues are embedded in this culture gel layer to culture the cell tissues. As a method for observing cultured cell tissues, a method in which an inverted microscope or an upright microscope is used to observe cell tissues from a lower side or upper side of a culture gel layer has been employed. In Patent Literature 3, a confocal laser scanning microscope is used to observe cells stained with a dye.

In a confocal laser scanning microscope, a focal point of a laser beam is tied to a cell by an objective lens, and autofluorescence of a fluorescent substance with which the cell at the focal point is labeled can be detected. For this reason, a three-dimensional image of a cell can be created by superimposing, in the z-direction, a two-dimensional image on a plurality of focal planes (xy planes).

In these studies in which conventional cell tissues are three-dimensionally cultured, only a bright-field image of a cell tissue from above or below can be obtained. It is therefore difficult to recognize a three-dimensional structure of a cell tissue in a bright-field image. Further, in the case of a cell tissue that is thick in its height direction, it is difficult to observe the cell tissue because light does not pass through a part that has a high cellular density.

Thus, in order to solve these problems, the inventors of the present invention have proposed a cell culture vessel/observation sample cell that makes it possible to three-dimensionally culture a cell or cell tissue and that makes it possible to easily understand a three-dimensional structure of the cultured cell or cell tissue by multifaceted observation (Patent Literature 4, etc.).

### Citation List

### [Patent Literature]

[Patent Literature 1]
   Japanese Patent Application Publication Tokukai No. 2009-213716
[Patent Literature 2]
   International Publication No. WO2004/084967
[Patent Literature 3]
   International Publication No. WO2012/147878
[Patent Literature 4]
   International Publication No. WO2017/094451

### Summary of Invention

### Technical Problem

The cell culture vessel disclosed in Patent Literature 4 makes it possible to carry out multifaceted observation, and thus has an advantage of making it possible to easily observe a three-dimensional structure of a cultured cell or cell tissue while the cultured cell or cell tissue is stored in a vessel. However, for example, in a case where a weak signal derived from a cell is imaged, it is sometimes necessary to carry out observation by preparing and sectioning a block for preparation of an observation section which block includes a sample (cultured cell or cell tissue).

In this case, the block for preparation of an observation section which block is to be sectioned has been taken out from the vessel. In a case where this block is used, it is difficult to understand whether the block is sectioned in an appropriate direction. Further, even observation of a section makes it difficult to understand a positional correspondence relationship between a sample during a step (cell culture step, etc.) prior to subjecting the sample to observation and the sample on the section. In particular, in a cell culture step, in many cases, a substance with a concentration gradient given is supplied to induce a cell aggregate (organoid, etc.) with orientation. Thus, it has been increasingly important to understand a positional correspondence relationship between a sample during a step prior to subjecting the sample to observation and a sample on a section.

The present invention has an object to provide, for example, a block for preparation of an observation section which block makes it easy to understand whether the block is sectioned in an appropriate direction and which block holds position information pertaining to an embedded sample.

### Solution to Problem

In order to attain the object, the present invention includes the following aspects.
<1> A block for preparation of an observation section, including: a cell aggregate that has a three-dimensional shape and that is embedded; and a marker that is continuous or intermittent and that extends in a predetermined direction.
<2> A jig for forming, in a gel filled in a cell culture vessel which is polyhedral and at least one surface of which is open, a gap in which cells are to be seeded, the jig including a surface having (i) a frame body that is shaped so as to fit in an outer circumference of the open at least one surface of the cell culture vessel and (ii) a protrusion that is located on an inner side of the frame body.

### Advantageous Effects of Invention

An aspect of the present invention makes it possible to provide, for example, a block for preparation of an observation section which block holds position information pertaining to an embedded sample.

### Brief Description of Drawings

Fig. 1 is a view for describing a process in accordance with an example of the present invention.
Fig. 2 is a view for describing a process in accordance with an example of the present invention.
Fig. 3 is a view for describing a process in accordance with an example of the present invention.
Fig. 4 shows images of a cube (vessel) and a concentration gradient chip in accordance with an example of the present invention.
Fig. 5 is shows images of observation sections in accordance with an example of the present invention.

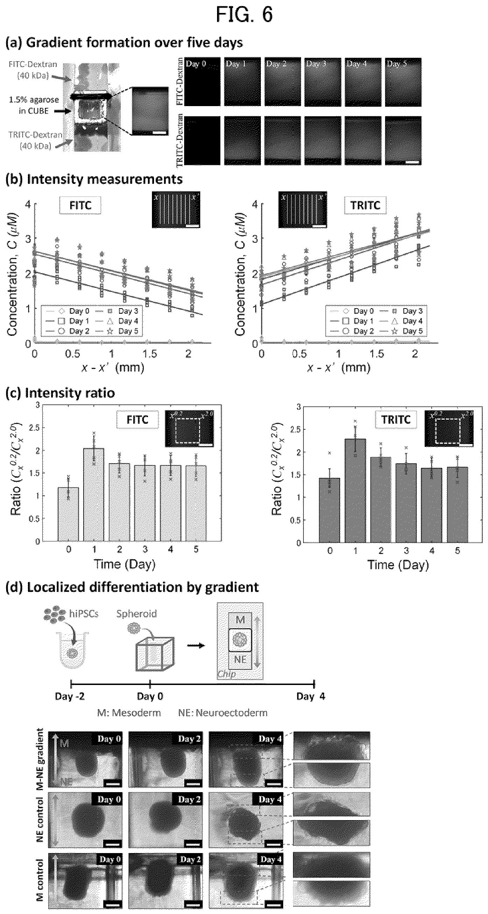

Fig. 6 is a view for describing generation of a concentration gradient and localized differentiation with use of a gradient-in-cube chip. Fig. 6(a) is a view obtained by imaging of FITC-dextran and TRITC-dextran concentration gradients formed over a 5-day period. Imaging was carried out every 24 hours after used dextran was removed, a medium chamber was washed with DPBS, and fresh dextran was added to the chamber. Dimensions of a window of a cube were 2.75 mm in width and 3.5 mm in height, and an imaging field of view at 4× magnification was 3.6 mm in width and a height of 2.7 mm. The scale bar is 1 mm. In Fig. 6(b), a concentration C was determined by linearly correlating an average intensity along the y-axis for each pixel in a center region (2.2 mm in width and 2.2 mm in height) of a fluorescence image from x to x', the average intensity having been obtained from a standard curve of FITC-dextran and TRITC-dextran concentrations. The scale bar is 1 mm. Markers represent selected data points at every 50 pixels (about 0.3 mm), and lines show the linear least squares best fit; n=5. Slopes of individual lines for FITC were - 0.0044 for day 0, -0.5586 for day 1, -0.5607 for day 2, -0.5549 for day 3, -0.5487 for day 4, and -0.5391 for day 5. Slopes for TRITC were 0.0095 for day 0, 0.7644 for day 1, 0.7062 for day 2, 0.6504 for day 3, 0.5897 for day 4, and 0.6039 for day 5. Day 0 shows a slight concentration gradient. For day 1, concentration gradients in opposite directions for FITC and TRITC, respectively, are formed from one end to an opposite end of the cube. Fig. 6(c) is a view obtained by taking a ratio between a source-end concentration and a sink-end concentration as an indicator indicating concentration gradient steepness. A region from x = 0.2 mm to x = 2.0 mm was analyzed and substantially corresponds to a region in which a spheroid would be located. The concentration gradient was the steepest on day 1. Although steepness of the concentration gradient decreased from day 2, the concentration gradient was kept constant for 5 days. Fig. 6(d) is a view illustrating a state of differentiation of hiPSC spheroid by action of a mesoderminducing medium (M) from one side and a neuroectoderminducing medium (NE) from an opposite side. Unlike M or NE only controls, the morphologies of which are substantially uniform, a spheroid, both ends of which have different morphologies, has been formed. The scale bar is 500 µm.

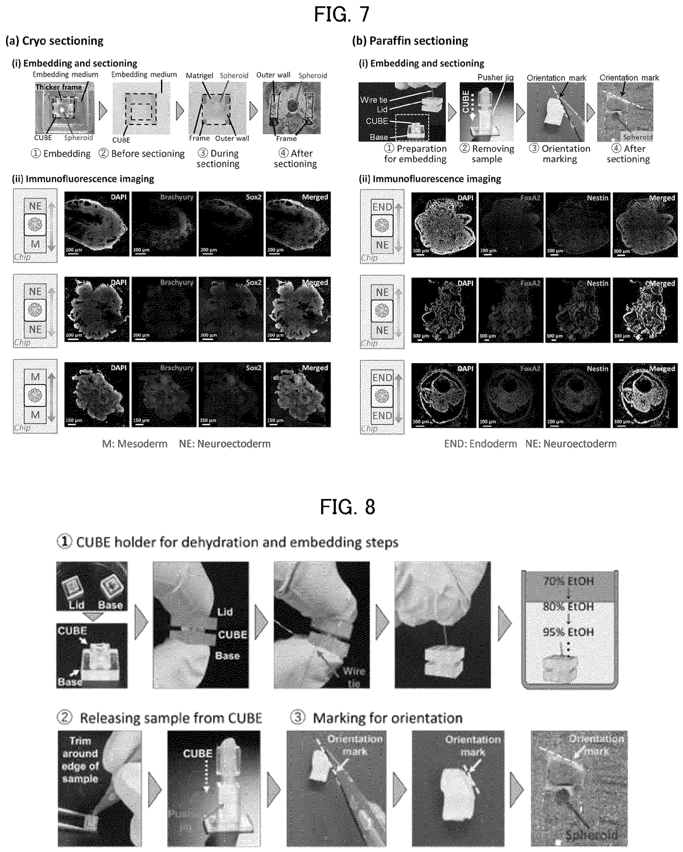

Fig. 7 illustrates immunostaining imaging after cryo sectioning and paraffin sectioning. (a) Cryo sectioning and imaging. (i) illustrates steps of embedding and sectioning frozen samples with a cube, and a cube frame was used as a reference marker for sample orientation. (ii) immunostaining of hiPSC spheroid differentiated by an M-NE concentration gradient showed a localized expression pattern of mesoderm (Brachyury) and neuroectoderm (Sox2) markers, whereas an NE only control and an M only control showed uniform distribution of the markers. (b) Paraffin sectioning and imaging. (i) illustrates steps of embedding and sectioning paraffin samples with use of a cube holder in order to prevent sample loss, and then a sample was removed from the cube, and an edge of the sample was cut in order to mark orientation. (ii) immunostaining of hiPSC spheroid differentiated by an endoderm (END)-NE gradient showed a localized expression pattern of endoderm (FoxA2) and neuroectoderm (Nestin) markers, whereas an NE only control and an END only control showed uniform distribution of the markers.
Fig. 8 illustrates a detailed process showing preparation of a sample for paraffin sectioning. A cube holder was designed in order to prevent a risk of sample loss during a paraffinization process. After paraffinization, a sample was released into a cube, and an edge of the sample was cut in order to mark orientation.

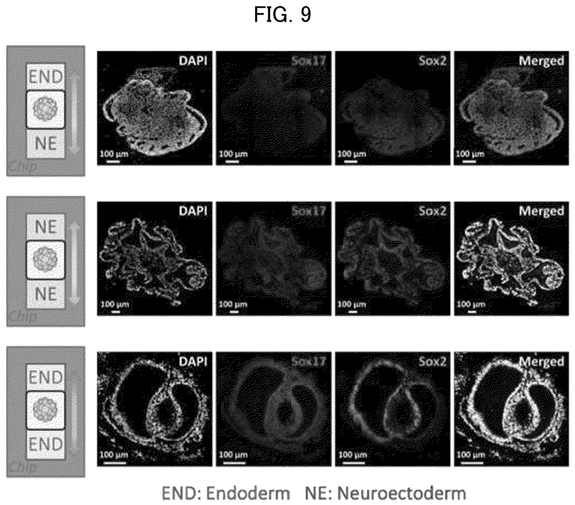

Fig. 9 illustrates immunofluorescence of a paraffin-sectioned sample of END-NE differentiated spheroid. Immunostaining showed a localized expression pattern of endoderm (Sox17) and neuroectoderm (Sox2) markers in END-NE spheroid, whereas a control (NE only) and a control (END only) showed uniform distribution of the markers.

### Description of Embodiments

### [1. Block for preparation of observation section]

Blocks 100 and 101 for preparation of an observation section in accordance with an embodiment of the present invention will be described in detail with reference to the drawings. Such a block includes (1) a cell aggregate that has a three-dimensional shape and that is embedded and (2) a marker that is continuous or intermittent and that extends in a predetermined direction.

### (Cell aggregate)

A cell aggregate 12b included in each of the blocks 100 and 101 is an object to be observed. A cell aggregate, which broadly refers to one that is an aggregate of a plurality of cells and that has a three-dimensional shape, is preferably a tissue or partial tissue of an organism, or a cell aggregate prepared in vitro by a method such as culture, and is more preferably an organoid. The organoid is also referred to as a mini-organ that has been three-dimensionally formed in vitro. The organoid is exemplified by, but not particularly limited to, a tongue organoid, a lung organoid, a gastric organoid, an intestinal organoid, a prostate organoid, a liver organoid, a pancreatic organoid, a kidney organoid, a mammary organoid, a thyroid organoid, a thymic organoid, a brain organoid, an inner ear organoid, and an epithelial organoid.

The size of the cell aggregate is not particularly limited. However, from the viewpoint that orientation of the cell aggregate is difficult to visually recognize and that the present invention is more suitable to be applied to the cell aggregate, the size is, for example, in a range of 10 mm or less, and is sometimes preferably in a range of 5 mm or less. The size of the cell aggregate has a lower limit that is not particularly limited. For example, the lower limit is sometimes preferably in a range of 1 mm or more.

The cell aggregate included in a block is, for example, assigned with a label for observation. Examples of the label for observation include (1) labeling by expression of a fluorescent protein or a photoprotein and (2) labeling by a fluorescent dye. Note that the cell aggregate may be labeled by a method such as immunostaining after being sectioned.

The cell aggregate included in the block is embedded. Embedding has a meaning used in the field of microscopic observation, i.e., means that a target object such as a cell aggregate containing much moisture is solidified so that the target object can be sectioned. Embedding is exemplified by, but not particularly limited to, methods such as paraffin embedding, resin embedding, and cryo embedding.

### (Marker)

A marker included in each of the blocks 100 and 101 is a marker that is continuous or intermittent and that extends in a predetermined direction. The marker appears on each section in a case where the block is sectioned in a plane intersecting the predetermined direction. In a case where the block is sectioned in an appropriate direction, the marker appears on each section together with the cell aggregate.

This marker makes it easy to understand whether the block is sectioned in an appropriate direction, and further can be used as position information pertaining to a sample (cell aggregate) that is embedded in the block. Specifically, using the marker makes it possible to understand a positional correspondence relationship between a sample during a step (cell culture step, etc.) prior to subjecting the sample to sectioning or observation and the sample on a section. That is, it can be said that the marker indicates information pertaining to a direction of a process that the cell aggregate or a precursor thereof has undergone.

The marker is exemplified by, but not particularly limited to, the following.
(1) The block is in a form of being stored in a vessel, and all or part of the vessel serves as the marker. The cryo-embedded block 101 in Examples (described later) corresponds to this, and a frame 10a or a PDMS sidewall 10b of a cube, the frame 10a or the sidewall 10b being a component of the vessel, serves as the marker.
(2) The block includes a notch extending in the predetermined direction, the notch serving as the marker. The paraffin-embedded block 100 in Examples (described later) which has been taken out from the vessel (cube) and one edge (side) of which has been cut off corresponds to this, and a cut-off part 110 serves as the marker.
(3) A structure embedded in the block in the predetermined direction, a small hole provided in the block in the predetermined direction, or the like.

Note that the vessel of (1) or the structure of (3) needs to be made of a material which allows the vessel or the structure to be sectioned. The vessel of (1) is, for example, a vessel (cube) made of polycarbonate or a vessel (cube) made of Teflon (trademark, the same hereinafter). In contrast, the vessel (cube) of (2) is desired to have high tolerance to an organic solvent in a paraffin embedding process, and is, for example, a vessel (cube) made of aluminum or a vessel (cube) made of Teflon.

### (Shape of block, etc.)

The block has a shape that is not particularly limited and that is, for example, a polyhedral shape, and is preferably a rectangular shape such as an approximately rectangular parallelepiped shape or an approximately cubic shape. In the case of a rectangular shape, the length of a side is not particularly limited and is, for example, approximately 1 mm to 10 mm, and preferably approximately 3 mm to 5 mm. The block is for preparation of an observation section and refers to a block in a state of being able to be set in, for example, a microtome as it is so as to be sectioned.

### [2. Method for preparing block for preparation of observation section]

A block for preparation of an observation section is prepared, for example, through the following steps:
(1) a step A of carrying out a predetermined process with respect to a cell aggregate that is stored in a vessel or a precursor of the cell aggregate;
(2) a step B of embedding the cell aggregate that is stored in the vessel and that has been subjected to the step A; and
(3) a step C of taking out, from the vessel, a block including the cell aggregate embedded in the step B, and forming a notch (marker) for the block taken out, the step C being carried out as necessary.

### (Step A)

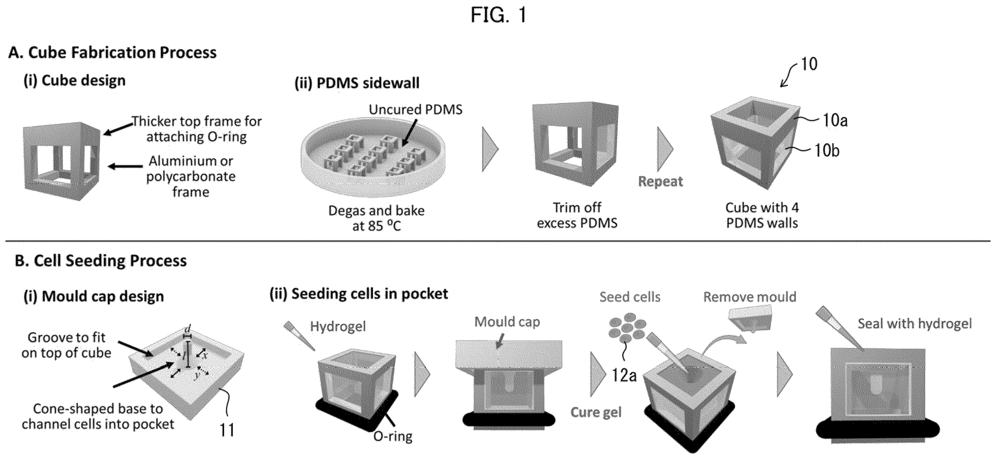

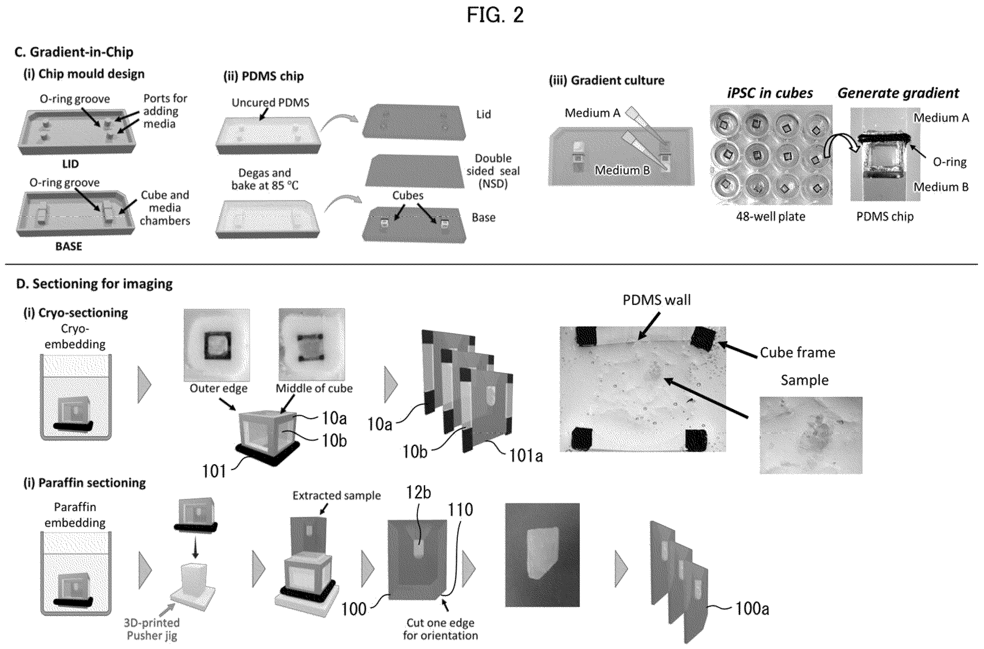

A vessel 10 that stores a cell aggregate or a precursor 12a thereof is exemplified by, but not particularly limited to, vessels illustrated in Figs. 1 and 2. The vessel includes a frame part (frame) that has a polyhedral shape and a lighttransmissive window part that covers a surface surrounded by the frame part. The vessel 10 illustrated in Figs. 1 and 2 includes a frame part (frame 10a) that has a substantially cubic shape and a window part (sidewall 10b) that is provided on each of four surfaces among six surfaces each surrounded by the frame part. Note that the window part may be made of not only PDMS shown in Examples but also any of hydrogels such as an agarose gel and a polyacrylamide gel. Note also that the vessel can be any of those disclosed in, for example, WO2017/094451, WO2018/150689, and WO2022/080162.

An inner side of the vessel is filled with a culture gel for culture of a cell aggregate or a precursor thereof. Note that a gap in which a cell aggregate or a precursor thereof is to be disposed can be formed with high repeatability by filling a vessel with a culture gel before curing and then covering the vessel with a mold cap 11 (shown in Examples) so as to cure the culture gel.

That is, the mold cap is "a jig for forming, in a gel filled in a cell culture vessel which is polyhedral and at least one surface of which is open, a gap in which cells are to be seeded, the jig including a surface having (i) a frame body that is shaped so as to fit in an outer circumference of the open at least one surface of the cell culture vessel and (ii) a protrusion that is located on an inner side of the frame body". The frame body precisely defines a positional relationship between the cell culture vessel and the mold cap, and the protrusion makes it possible to form, in a culture gel with high repeatability, a gap having a predetermined depth.

Note that the precursor 12a of the cell aggregate refers to a precursor which is to be a cell aggregate by being cultured in a culture gel. Examples of such a precursor include ES cells, pluripotent stem cells such as an iPS cell, and other stem cells.

A predetermined process carried out with respect to a cell aggregate or a precursor thereof in the step A is not particularly limited in type. Examples of the predetermined process include culture with supply of a substance and a chemical agent treatment. Examples of the culture with supply of a substance include induction of a cell aggregate with use of a morphogen concentration gradient (shown in Examples). Examples of the chemical agent treatment include a chemical agent metabolism experiment and a chemical agent (candidate) screening experiment. The predetermined process is, for example, a process with orientation with respect to a cell aggregate or a precursor thereof (relatively also to a vessel), and the above-described process in which a concentration gradient of a substance is used is an example of the process. Note that, unless a substance is supplied uniformly to a cell aggregate or a precursor thereof in any direction, a process with supply of a substance falls under the process with orientation. A device for carrying out the step A is, for example, a cell culture fluidic chip disclosed in WO2018/147032. According to this cell culture fluidic chip, a vessel storing a cell aggregate or a precursor thereof is set as it is so as to carry out a process, and the vessel can be removed after the process.

### (Step B)

The step B is a step of embedding the cell aggregate that is stored in the vessel and that has been subjected to the step A. An embedding method such as paraffin embedding, resin embedding, or cryo embedding need only be a method known in the field of preparation of an observation section. Through this step, a cryo-embedded block for preparation of an observation section illustrated in Fig. 2 is produced.

### (Step C)

The step C is carried out as necessary. For example, in production of paraffin-embedded block for preparation of an observation section (see Fig. 2), a block including the cell aggregate embedded in the step B is taken out from the vessel, and a notch (marker) is formed for the block taken out.

In the block for preparation of an observation section which block has been prepared in the step B, all or part of the vessel serves as the marker. Further, in the block for preparation of an observation section which block has been prepared in the step C, the notch formed for the block serves as the marker. Carrying out the steps A and B or the steps A to C while understanding a correspondence relationship between a direction of a process carried out in the step A and each marker results in indication of information pertaining to a direction of a process that the cell aggregate or a precursor thereof has undergone.

### [3. Method for preparing section from block for preparation of observation section, method for observing section, etc.]

This method is a method including slicing, in a direction intersecting the predetermined direction (that is, a direction in which the marker extends), the block described in the [1. Block for preparation of observation section] section, and preparing observation sections 100a and 101a. This makes it possible to prepare a section for observation on which section a marker and a cell aggregate are placed. A method for preparing a section from a block can be applied as it is to a method for preparing a section for microscopic observation. For example, the section need only be made with the block set in a microtome.

Note that a step of using a microscope to observe a prepared observation section need only be in accordance with a known observation method. Note also that the microscope is not particularly limited in type provided that the microscope is an optical microscope. For example, the microscope may be either an upright microscope or an inverted microscope. An observation method may be, for example, bright-field observation, dark-field observation, phase contrast observation, differential interference observation, HMC observation, or polarized light observation. The observation method may be fluorescence microscopic observation with use of, for example, an epifluorescence microscope, a total internal reflection fluorescence microscope, a confocal laser fluorescence microscope, or a multiphoton excitation fluorescence microscope.

The marker is placed on the prepared observation section. This marker indicates information pertaining to a direction of a process that a cell aggregate or a precursor thereof has undergone, and can be referred to in observation of each section. Further, observation of how the marker is cut is also helpful in confirming whether the block is sectioned in an appropriate direction. Furthermore, alignment with use of the marker can be used to reconstruct one stacking image from images of a plurality of sections.

### (Recap)

The present invention includes, for example, the following invention.
<1> A block for preparation of an observation section, including: a cell aggregate that has a three-dimensional shape and that is embedded; and a marker that is continuous or intermittent and that extends in a predetermined direction.
<2> The block described in <1>, wherein the block is cryo-embedded or paraffin-embedded.
<3> The block described in <1>, wherein the block is in a form of being stored in a vessel, and all or part of the vessel serves as the marker.
<4> The block described in <1>, further including a notch extending in the predetermined direction, the notch serving as the marker.
<5> The block described in <1>, wherein the cell aggregate is an organoid.
<6> The block described in <1>, wherein the marker indicates information pertaining to a direction of a process that the cell aggregate or a precursor thereof has undergone.
<7> A method for preparing a section, including slicing, in a direction intersecting the predetermined direction, the block described in any one of <1> to <6>, and preparing an observation section.
<8> An observation section prepared by the method described in <7>.
<9> An observation method including using an optical microscope to observe the observation section described in <8>.
<10> A method for preparing the block described in any one of <1> to <6>, the method including: a step A of carrying out a predetermined process with respect to a cell aggregate that is stored in a vessel or a precursor of the cell aggregate; and a step B of embedding the cell aggregate that is stored in the vessel and that has been subjected to the step A.
<11> A jig for forming, in a gel filled in a cell culture vessel which is polyhedral and at least one surface of which is open, a gap in which cells are to be seeded, the jig including a surface having (i) a frame body that is shaped so as to fit in an outer circumference of the open at least one surface of the cell culture vessel and (ii) a protrusion that is located on an inner side of the frame body.
< 12> An organoid evaluation method including: a first step of, during generation of an organoid by culture of cells in a culture vessel, generating the organoid with orientation by providing a concentration gradient; and a second step of, during embedding and sectioning of the organoid with orientation, which has been generated in the first step, sectioning the organoid while position information is held.
<13> In <12>, in the second step, a section holds the position information by sectioning the culture vessel concurrently with embedding and sectioning the organoid.
<14> In <12>, in the second step, a section holds the position information by carrying out processing for removing part of an embedded block including the organoid that has been taken out from the culture vessel, and then sectioning the organoid.

### Examples

The following description will discuss an example of the present invention in detail.

### <Culture of hiPSC>

On dishes coated with 0.5 µg/cm² Laminin-511 E8 (iMatrix-511 Silk; Nippi, 892021), human iPSCs (1231A3; RIKEN BRC Cell Bank, HPS0381) were cultured with use of StemFit AK02N (Ajinomoto Co., Inc., RC AK02N) and passaged with use of ReLeSR (STEMCELL Technologies, 05872).

### <Fabrication of cube (vessel)>

Aluminum cube frames having a width of 5 mm were designed with use of Rhinoceros 3D software and ordered from a machining company (Proto Labs, Japan). A cube was designed with a thicker frame on a top side of the cube so that an O-ring can be attached to the cube (Fig. 1A(i)). The O-ring is for further ensuring sealing between the cube and a PDMS chip in order to reduce leakage of media around the cube during concentration gradient culture. In order to form a plurality of sidewalls (windows) of the cube, first, uncured polydimethylsiloxane (PDMS; Silpot 184, Dow Toray, 04133124) was prepared by mixing an elastomer base with a curing agent at a ratio of 10: 1. Then, a thin layer of the mixture (uncured PDMS) was spread out in a Petri dish and degassed in order to remove air bubbles. A cube frame was placed on the uncured PDMS, then degassed again, and baked at 85°C for 15 minutes to cure the PDMS. Upon being cured, the PDMS was cut from the frame and trimmed with use of a scalpel, and this process was repeated to also cover openings on the other three side surfaces of the cube with the sidewalls of the cured PDMS, so that the cube having an open top surface and an open bottom was prepared (Fig. 1A(ii)). The cube having four PDMS sidewalls was washed twice by ultrasonication, washed once with MilliQ water, and further washed once with isopropanol, and then dried in an oven. For sterilization, the cube was autoclaved and then dried in an oven before being used.

### <hiPSC seeding in cube>

In order to control positions of cells during initial seeding, a mold cap was designed, the mold cap including: (1) a columnar structure which has a conical base and which reaches a desired seeding position in the cube; and (2) a groove which ensures that the groove fits on the top surface of the cube so that a position of the columnar structure is properly adjusted in the cube (Fig. 1B(i)). Before being used, the mold cap was coated with 2-methacryloyloxyethylphosphorylcholine (MPC; Lipidure, NOF Corporation, CM5206E) diluted to 5% in isopropanol, and dried for 1 hour at room temperature (RT). A nitrile O-ring (AS ONE, 62-3049-63) was attached to a thick part of an aluminum frame of the cube, and the cube was placed in a dish with the O-ring side facing downward. Matrigel (registered trademark) (Corning, 356231) was added into the cube. Then, the mold cap was placed on the top surface of the cube, and the Matrigel was cured in an incubator (37°C, 5% CO₂) for 25 minutes. During this period, Accutase (registered trademark) (Invitrogen, 00455556) was used to separate hiPSC cells and centrifuge the hiPSC cells at 200 ×g for 3 minutes at room temperature so as to form the hiPSC cells into a pellet, and a supernatant was discarded. As soon as the Matrigel was cured, the mold cap was removed, and the cells were seeded in a pocket produced by a mold.

From two 35 mm dishes or one 60 mm dish, 1 µL of cells formed into a pellet were seeded in one cube. The cube was returned to the incubator for 5 minutes so as to fix the cells in the pocket. Next, additional Matrigel (registered trademark) was added to the top surface of the cube and cured for 25 minutes so as to seal the pocket (Fig. 1B(ii)). After curing, the cube was transferred to a 48-well plate having a 1 mL StemFit medium and a 10 µM Rock inhibitor (Y-27632; Nacalai Tesque, 08945-84). On the following 2 days, the medium was switched to a StemFit medium without Y-27632.

### <hiPSC differentiation with use of concentration gradient formed in chip>

hiPSCs were seeded in a cube, and, after it was confirmed that the hiPSCs had been aggregated to form a spheroid, a sample was transferred to a chip in which a concentration gradient is formed, and localized differentiation was started. A mold for making a lid of a PDMS gradient chip was designed to include a groove that fits in an O-ring and a plurality of ports for adding media, and a mold for making a base of the PDMS gradient chip was designed to fit in the cube, the O-ring, and two separate media chambers (Fig. 2C(i)). In order to make PDMS chips, uncured PDMS was poured into the above two molds, and then degassed and baked at 85°C for 30 minutes so as to cure the PDMS. As soon as the PDMS was cured, two chips formed were taken out from the molds, and then washed and sterilized in the same way as in the case of the cube. In order to assemble a concentration gradient chip, a sample cube provided with the O-ring was placed on the base side of the chip, and then a PDMS adhesive film (NSD-100, NIPPA) was used to combine and seal the lid and the base together (Fig. 2C(ii)). After assembly, one side and its opposite side of the two media chambers were filled with a STEMdiff Trilineage Ectoderm medium (STEMCELL Technologies, 05231) and a STEMdiff Trilineage Endoderm medium (STEMCELL Technologies, 05233), respectively (Fig. 2C(iii)). Media replacement was carried out by discarding consumed media, and washing was carried out once with DPBS before replacement with fresh media. Specifically, a morphogen concentration gradient has been formed between two surfaces without PDMS sidewalls of the cube.

### <Induction of differentiation>

A neuroectoderm differentiation medium (NE medium) contained a 1:1 mixture of KnockOut DMEM/F12 (Gibco, 12660-012) and a Neurobasal (trademark) medium (Gibco, 21103-049) supplemented with 10% KnockOut serum replacement (Gibco, 10828010), 1% MEM non-essential amino acid (Nacalai Tesque, 06344-56), 1% GlutaMAX (Gibco, 35050-061), 1µM LDN1913189 (Sigma, SML0559), 2pM SB431542 (Nacalai Tesque, 18176-54), 3µM CHIR99021 (Nacalai Tesque, 18764-44), 0.1 mM 2-mercaptoethanol (Nacalai Tesque, 21438-82), and 0.5 µM ascorbic acid (Nacalai Tesque, 03420-52). A mesoderm basal medium contained RPMI medium 1640 (Gibco, 11875-093), 1% GlutaMAX, and 1% penicillin-streptomycin (Gibco, 15140122). For mesoderm differentiation, 5 µM CHIR99021 was added to the mesoderm basal medium on days 0 and 1. On days 2 to 4, CHIR99021 was withdrawn and replaced with 100 ng/mL bFGF (Nacalai Tesque, 19155-36) and 1 µM all-trans retinoic acid (Stemgent, 04-0021). For endoderm differentiation, 5 µM CHIR99021 was added to the mesoderm basal medium on day 0. On days 2 to 5, CHIR99021 was withdrawn and replaced with 100 ng/mL Activin A (R&D Systems, 338-AC-050/CF). Media replacement was carried out every day by discarding used media, carrying out washing once with DPBS, and then carrying out replacement with fresh media. In order to observe a spheroid morphological change in a cube, an Olympus CKX41 microscope was used to obtain a spheroid phase-contrast image.

### <Fixation and sectioning for imaging>

When a sample was made analyzable, a sample cube was taken out from a concentration gradient chip and transferred to a 48-well plate for fixation. The sample cube as a whole was washed twice with DPBS for 5 minutes each time, fixed with 4% paraformaldehyde for 20 minutes, and then washed twice with DPBS for 10 minutes each time. For cryo sectioning, a PTFE cube was used instead of an aluminum cube, and, after fixation, a sample was embedded in a cryo sectioning embedding solution (White Tissue-Coat, Yuaikasei). After being frozen, the sample was sectioned together with the cube (Fig. 2D(i)). In cryo sectioning, a direction in which two sides with only a frame of the cube and without a PDMS sidewall are connected matches a direction of a morphogen concentration gradient during hiPSC differentiation induction.

For paraffin sectioning, the sample in the cube was embedded in paraffin by the following processing procedure: 70% ethanol for 30 minutes twice, 80% ethanol for 1 hour, 95% ethanol for 1 hour, 95% ethanol for 1 hour, 99% ethanol for 1 hour, 100% isopropanol for 1 hour twice, a mixture of isopropanol and xylene (volume ratio: 7:3) for 1 hour, a mixture of isopropanol and xylene (volume ratio: 1:1) for 1 hour, a mixture of isopropanol and xylene (volume ratio: 3:7) for 1 hour, 100% xylene for 1 hour, and paraffin for 1 hour twice.

Subsequently, after the cube was cut out from the paraffin, the sample (paraffin block) was removed from the cube by using a scalpel to cut the paraffin along an inner side of the frame and pressing the cube onto a pusher jig (Fig. 2D(ii)). Concentration gradient orientation was marked by cutting an edge (side) of the sample (paraffin block), and then the sample was cut into a plurality of slices.

The slices obtained were deparaffinized by the following method: 100% xylene for 10 minutes twice, 99% ethanol for 5 minutes twice, 95% ethanol for 5 minutes, 80% ethanol for 5 minutes, 70% ethanol for 5 minutes, and MilliQ water for 5 minutes. Heat-induced antigen retrieval was carried out by placing the sample in a boiling 10 mM pH6 citrate buffer for 10 seconds, then cooling the sample for 10 seconds, and repeating such boiling and cooling 6 times. During cooling of the sample, the sample was washed with MilliQ water for 5 minutes and then washed 3 times with DPBS for 5 minutes. In a section of a paraffin-embedded block, while a cut-off part is located at the bottom right, a vertical direction of the section matches a direction of a morphogen concentration gradient during hiPSC differentiation induction.

### <Immunostaining>

Cryo-sectioned samples were permeabilized with 0.5% Triton (trademark) X-100 for 10 minutes and then washed 3 times with 100 mM glycine for 10 minutes each time. An immunofluorescence buffer (IF buffer) was prepared by causing DPBS to contain 0.5% Tween (trademark) 20, 2% Triton X-100, and 10% bovine serum albumin (BSA; Sigma, 126615). Blocking was carried out by incubating the samples for 30 minutes with IF (IF+G) containing 10% goat serum (Gibco, 16210064), and then incubating the samples for 20 minutes with IF+G containing 1% goat anti-mouse IgG (Bethyl Laboratories, A90-116A). Antibodies were diluted (1:200 or 1 µg/mL) in accordance with Table 1 below. Primary antibodies were incubated for 90 minutes, and secondary antibodies (Alexa fluor, 1:200, Thermo FisherScientific) were incubated for 50 minutes. After incubation with each antibody, the samples were washed 3 times with an IF buffer for 15 minutes each time. Nuclei were stained with DAPI for 20 minutes and then washed 3 times with DPBS for 5 minutes each time.

**[Table 1]**

| Table 1. List of antibodies for immunofluorescence staining | | |
|---|---|---|
| **Reagents** | **Source and Identifier** | **Dilution** |
| Mouse monoclonal Anti-Sox2 | Abcam, ab79351 | 1:200 in IF+G |
| Rabbit monoclonal Anti-Nestin | Abcam, ab105389 | 1:200 in IF+G |
| Goat polyclonal Anti-Brachyury | R&D Systems, AF2085 | 1 µg/mL in IF+0.1% BSA |
| Goat polyclonal Anti-FoxA2 | R&D Systems, AF2400 | 1 µg/mL in IF+0.1% BSA |
| Goat polyclonal Anti-Sox17 | R&D Systems, AF1924 | 1 µg/mL in IF+0.1% BSA |
| Donkey anti-goat AF555 | Invitrogen, A32816 | 1:200 in IF+0.1% BSA |
| Goat anti-mouse AF488 | Invitrogen, A32723 | 1:200 in IF+G |
| Goat anti-rabbit AF488 | Invitrogen, A32731 | 1:200 in IF+G |
| DAPI | Invitrogen, D1306 | 600 nM in DPBS |

### <Examples>

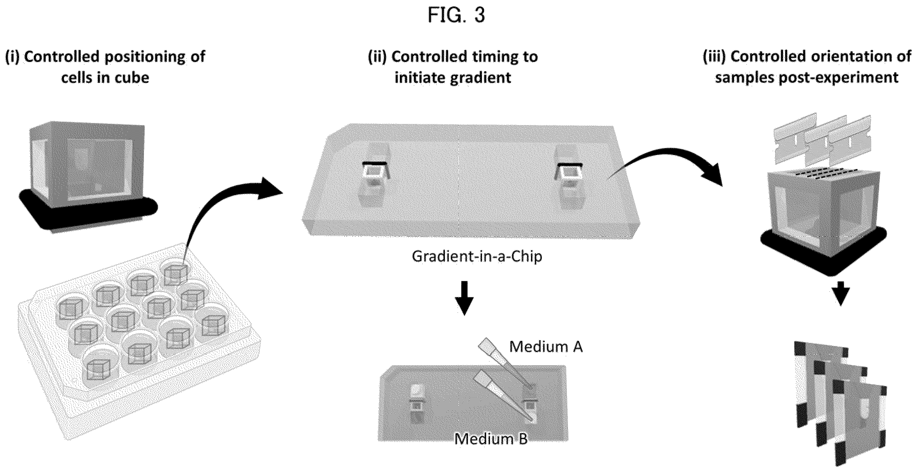

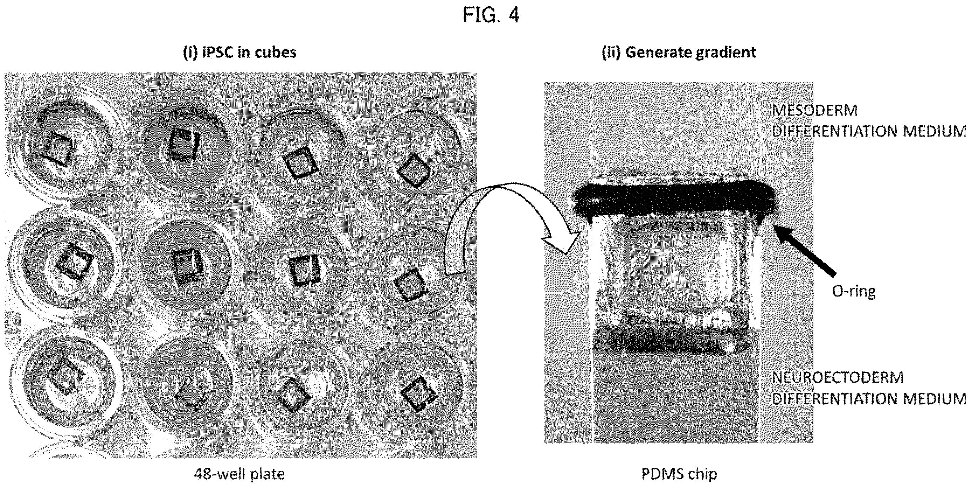

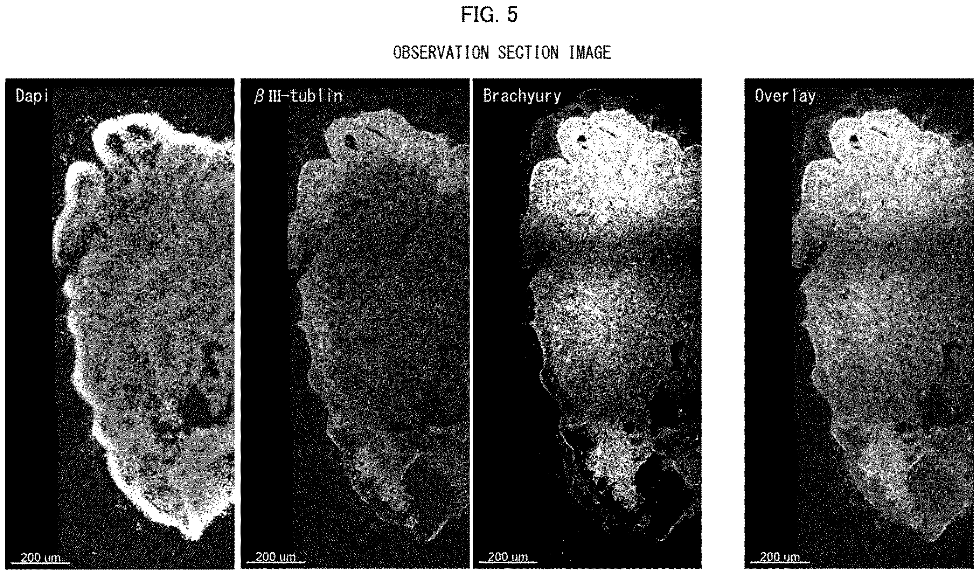

A cube that makes it possible to control a cell position was prepared by the foregoing method, hiPSCs were seeded, and the cube was transferred to a 48-well plate (Figs. 3(i) and 4(i)). Subsequently, a sample was transferred to a chip in which a concentration gradient is formed with use of a mesoderm differentiation medium and a neuroectoderm differentiation medium (NE medium), and 5-day culture was carried out (Figs. 3(ii) and 4(ii)). When the sample was made analyzable, the sample cube was taken out from the concentration gradient chip, and a cryo-section was prepared by the foregoing method (Fig. 3(iii)). Fig. 5 illustrates images of cryo-sections. In Fig. 5, the left three images label Dapi, β III-tublin, and Brachyury, respectively, and the rightmost image is an overlay image of each image. Further, in the images of Fig. 5, a gradient of the mesoderm differentiation medium is formed from above, and a gradient of the neuroectoderm differentiation medium is formed from below.

### <Concentration gradient formation and localized differentiation with use of gradient-in-cube chip>

For the purpose of simulating addition of two different types of media to the cube, FITC-dextran and TRITC-dextran were used to carry out daily imaging over a 5-day period in order to monitor the progress of formation of two concentration gradients in the cube (Fig. 6(a)).

Average concentrations (C) of FITC and TRITC across a center region (x-x') of a window of the cube were measured. By movement of FITC-dextran molecules and TRITC-dextran molecules from a high-concentration side to an opposite lowconcentration side (Fig. 6(b)), FITC and TRITC showed opposing concentration gradients with a high concentration on the source side and a low concentration on the sink side.

To a source reservoir, 10 µM of dextran was added, and an average maximum concentration at a source end of FOV was 2.618 µM for FITC, and 3.255 µM for TRITC, whereas an average minimum concentration at a sink end of the FOV was 0.024 µM for FITC, and 0.026 µM for TRITC.

The concentration gradient was calculated as a ratio of Iₓ^{0.2}/Iₓ^{2.0} for FITC and calculated as a ratio of Iₓ^{2.0}/Iₓ^{0.2} for TRITC, where one shows no gradient, and a value indicating a higher ratio represents a steeper gradient (Fig. 6(c)).

As the molecules entered a gel, over the first 24 hours, steepness of the concentration gradient increased and peaked. However, as the molecules accumulated on the opposite side as well as in the gel in the cube, the steepness of the concentration gradient decreased by day 2. Despite daily washing with DPBS and replacement with fresh dextran, a constant concentration gradient was successfully maintained over a 5-day period without the media reaching a state of equilibrium.

Here, only diffusion of 40 kDa dextran into an agarose gel was used to model diffusion of various growth factors into an ECM hydrogel. The reason why these were chosen is based on the fact that a molecular weight of Wnt, which plays an important signaling role in development, is approximately 40 kDa and that dextran and agarose are readily available reagents which have been long used in the fields of mass transport and diffusion studies. Given that a concentration gradient across a scale of the length of a single cell or a few cells is sufficient to provide position information to cells (Vetter, R. & Iber, Nat. Commun. 13, 1145 (2022); Matos, I. et al. Elife 9, e54304 (2020)), the inventors of the present invention postulated that the concentration gradient formed in the gradient-in-cube chip should be sufficient to induce localized differentiation at opposite ends of a spheroid. Additionally, another strategy for counteracting accumulation of morphogen is to supply a morphogen inhibitor from the opposite side of the concentration gradient. Morphogen and an inhibitor thereof are critical for patterning in tissues in vivo. For example, Dkk1, Lefty-1, and Cer-1 at an anterior end of an embryo, which are Wnt and Nodal agonists, restrict Wnt/Nodal to a posterior end of the embryo to form an anterior-posterior axis (Carlson, B. Human Embryology and Developmental Biology (Elsevier, 2018)). For the purpose of demonstrating application of the gradient-in-cube chip, in order to use the gradient-in-cube chip to differentiate a single spheroid into two localized regions, a neuroectoderm differentiation medium (NE) was supplied from one end of the cube, and a mesoderm differentiation medium (M) was supplied from the opposite side. The mesoderm differentiation medium (based on a protocol by Lam, A. Q. et al. J. Am. Soc. Nephrol. 25, 1211-1225 (2014).) contained a Wnt activator CHIR99021 at a high concentration, whereas the neuroectoderm differentiation medium (based on a protocol by Bianchi, F. et al. Stem Cell Res. 32, 126-134 (2018).) contained a lower-concentration Wnt activator and a Nodal/Activin inhibitor SB431542 to counteract mesoderm differentiation on the neuroectoderm side. After 4 days of differentiation, morphological changes were observed at both ends of the spheroid. As compared with the neuroectoderm side having a feature of being smoother, the mesoderm side had a feature of being bumpier and more protruding.

In contrast, control spheroids showed rather uniform morphological features. That is, a mesoderm control had bumpy protrusions all over the surface thereof, whereas a neuroectoderm control had a smoother surface (Fig. 6(d)).

### <Maintaining orientation during sectioning and imaging>

It is often difficult to visualize expression of cell pluripotency or differentiation markers in large-scale 3D samples such as organoids due to a limited range of laser penetration as well as a low sensitivity of low magnification objective lenses and short focal lengths of high-sensitivity lenses. Hence, organoids are sometimes embedded in a cryo medium or paraffin and sliced into thin sections for staining and imaging. However, orientation of a sample is often lost after the sample is retrieved from prevailing gradient-forming devices. A cube device has not only an advantage of causing a cube to contain therein cells so as to make it possible to retrieve the cells without causing any damage to a sample, but also an advantage of making it possible to easily mark orientation of a gradient so that the orientation can be recognized later. The following experiment shows that samples in a cube can be processed for cryo and paraffin sectioning.

For cryo sectioning, one frame of a cube is regarded as a thicker frame, and an attached O-ring acts as an orientation marker (Fig. 7(a)(i)). After a sample is retrieved from a gradient-in-cube chip, the cube can simply be soaked in sucrose and a cryo embedding medium before freezing. Once frozen, the cube is sliced together with the sample, and the cube frame and a PDMS outer wall act as a reference to maintain sample orientation (Figs. 2D(i) and 7(a)). From immunofluorescence staining of a hiPSC spheroid differentiated with an NE-M gradient, localization of a neuroectoderm marker Sox2 and a mesoderm marker Brachyury was observed on both sides of the spheroid, whereas both the markers were expressed uniformly throughout the spheroid in control samples (Fig. 7(b)). This result demonstrated a method in which orientation information of a spheroid differentiated by a morphogen concentration gradient is maintained by sectioning the sample as it is in the cube. However, a downside of this method is that a microtome blade is damaged by repeated cutting of a hard acrylic material and may need to be replaced frequently. This problem can be solved by using a material that makes it easy to cut the cube frame, such as polycarbonate or Teflon. Alternatively, a microtome blade or diamond-coated blade that is commonly used to cut a hard material such as a bone can be used to cut the cube frame.

For paraffin sectioning, some extra steps have to be taken in order to maintain integrity and gradient orientation of the sample in the cube (Figs. 2D(ii), 7b(i), and 8). While the cube can be sectioned by a cryo cube made of a combination of an acrylic frame and a PDMS wall, which is a soft material, the whole cube is made of acrylic, and a modification is necessary because PDMS is not compatible with an organic solvent used in a paraffin embedding process. This makes it very difficult to section a paraffin cube. During the initial dehydration process prior to paraffin embedding, a hydrogel loses a lot of volume and shrinks in the cube. In order to avoid the risk of losing the sample that may be detached from the cube, a cube holder including a lid and a base was designed to cover most of the top surface and the bottom open surface of the cube but still allow a reagent to pass through in and out of the sample. Cubes in the holder were tied with a wire to maintain the cubes in the holder. After a paraffinization process, a pusher jig was used to remove the sample from the cube, and the orientation was marked by cutting an edge at one corner of the sample. In order to demonstrate application to a paraffin method, hiPSC spheroid on one side of the cube was differentiated with NE and endoderm (END) differentiation (see a protocol by Lam, A. Q. et al. J. Am. Soc. Nephrol. 25, 1211-1225 (2014)) media. Immunostaining of neuroectoderm markers Nestin and Sox2 and endoderm markers FoxA2 and Sox17 showed that NE and END were each localized at both ends of the spheroid (Figs. 7(b)(ii) and 9). In contrast, control samples without concentration gradient culture showed uniform expression of markers throughout the spheroid.

### <Recap>

An object of the present Examples is to develop a simple concentration gradient culture platform for controlling differentiation of PSCs into organoids having desired patterns, and also to hold sample integrity and orientation information of a concentration gradient during culture for subsequent image analysis experiments. In order to achieve this, a previously developed cube culture device (Hagiwara et al., 2018a; Hagiwara et al., 2018b) was used to enhance handleability of samples cultured in an ECM hydrogel and repeatability of cell seeding and pattern formation. In the present Examples, it was shown first how to precisely position cells at desired locations in a cube culture device. Further, it has been demonstrated that a concentration gradient which induces dorsal-ventral differentiation of iPSCs could be generated in a cube merely by transferring the cube culture device to a two-compartment chip device. Finally, a concentration gradient platform involving a method that is a different post-experiment processing method and that is for carrying out imaging and analysis while maintaining orientation information of a concentration gradient is provided. With such a concentration gradient-in-cube platform, organoids with controlled body axis differentiation can be achieved. In order to further understand a developmental process in humans, it is also possible to provide more complex in vitro models that make it possible to systematically apply and study effects of morphogen concentration gradients in directing cell differentiation and development into tissues, organs, and eventually body systems.

Main unique points shown in the present Examples are summarized as follows: In order to achieve (1) a simple method, (2) strictly controlled placement or positioning of a sample in a device, and (3) further analysis after experiment, it is possible to retrieve a sample from a device without causing any major damage to the sample or losing orientation information of the sample.

### Industrial Applicability

The present invention makes it possible to provide, for example, a block for preparation of an observation section which block makes it easy to understand whether the block is sectioned in an appropriate direction and which block holds position information pertaining to an embedded sample. This makes it possible to, for example, carry out, all at once, generation, observation, sampling, evaluation, etc. of organoids in an organ-on-a-chip (organ chip) region that has attracted attention at the basic research stage and the nonclinical trial stage of development of pharmaceutical products.

### Reference Signs List

10 Cube (vessel)
10a Frame (all or part of vessel)
10b Sidewall (all or part of vessel)
11 Mold cap (jig)
12a Precursor of cell aggregate
12b Cell aggregate
100 Block (block for preparation of observation section)
100a Section
101 Block (block for preparation of observation section)
101a Section

## Claims

1. A block for preparation of an observation section, comprising:
a cell aggregate that has a three-dimensional shape and that is embedded; and
a marker that is continuous or intermittent and that extends in a predetermined direction.

2. The block as set forth in claim 1, wherein the block is cryo-embedded or paraffin-embedded.

3. The block as set forth in claim 1, wherein the block is in a form of being stored in a vessel, and all or part of the vessel serves as the marker.

4. The block as set forth in claim 1, further comprising a notch extending in the predetermined direction, the notch serving as the marker.

5. The block as set forth in claim 1, wherein the cell aggregate is an organoid.

6. The block as set forth in claim 1, wherein the marker indicates information pertaining to a direction of a process that the cell aggregate or a precursor thereof has undergone.

7. A method for preparing a section, comprising slicing, in a direction intersecting the predetermined direction, the block recited in any one of claims 1 to 6, and preparing an observation section.

8. An observation section prepared by the method recited in claim 7.

9. An observation method comprising using an optical microscope to observe the observation section recited in claim 8.

10. A method for preparing the block recited in any one of claims 1 to 6, said method comprising:
a step A of carrying out a predetermined process with respect to a cell aggregate that is stored in a vessel or a precursor of the cell aggregate; and
a step B of embedding the cell aggregate that is stored in the vessel and that has been subjected to the step A.

11. A jig for forming, in a gel filled in a cell culture vessel which is polyhedral and at least one surface of which is open, a gap in which cells are to be seeded,
said jig comprising a surface having (i) a frame body that is shaped so as to fit in an outer circumference of the open at least one surface of the cell culture vessel and (ii) a protrusion that is located on an inner side of the frame body.
